(19) Europäisches Patentamt / European Patent Office / Office européen des brevets

(11) **EP 4 682 540 A1**

(12) **EUROPEAN PATENT APPLICATION**
published in accordance with Art. 153(4) EPC

(43) Date of publication:
**21.01.2026   Bulletin 2026/04**

(21) Application number: **23927224.8**

(22) Date of filing: **21.12.2023**

(51) International Patent Classification (IPC):
**G01N 33/72** (2006.01)     **G01N 33/53** (2006.01)
**B01L 3/00** (2006.01)

(86) International application number:
**PCT/CN2023/140451**

(87) International publication number:
**WO 2024/187884 (19.09.2024 Gazette 2024/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **16.03.2023   CN 202310253439**

(71) Applicant: **Xue, Shan**
**Tianjin 300073 (CN)**

(72) Inventor: **Xue, Shan**
**Tianjin 300073 (CN)**

(74) Representative: **Bayramoglu et al.**
**Mira Office**
**Kanuni Sultan Süleyman Boulevard 5387**
**Street Beytepe, floor 12, no:50**
**06800 Cankaya, Ankara (TR)**

(54) **MICRO-FLUIDIC CHIP WITH MICRO-CHANNELS CAPABLE OF SELF-ADJUSTING HEIGHT THEREOF**

(57)    The present invention belongs to the field of in-vitro diagnostics and immunoassays, and particularly relates to a microfluidic chip with self-adjusting channel height. It comprises a substrate, liquid-guiding track, and top pressing column. The liquid-guiding track is made of a strip of flexible film which is placed on the upper surface of the substrate, and the natural gap between liquid-guiding track and the substrate forms a microchannel. One end of the track is designated as the terminal end, and the other end is the starting end which serves as the sample entrance. Between the track and the substrate, a labeling zone is located near the starting end, and a detection zone is set in the middle section of the track. The microchannel is open to atmosphere along both sides of its width, the channel height can self-adjust as liquid enters. The structure also includes a casing attached circumferentially to the substrate. The guiding track can be manufactured from rolled plastic film, the substrate from prefabricated plastic sheets, and the casing using standard injection molding processes. This avoids the need for precision machining, keeping costs low. The microchannel height is small, requiring only minimal sample volume, and adjusts automatically based on different sample properties, ensuring smooth fluid flow and broad applicability.

[FIG. 9]

**Description**

TECHNICAL FIELD

The invention pertains to the field of in-vitro diagnostics and immunoassays, particularly to a microfluidic chip with a microchannel height that can automatically adjust.

BACKGROUND TECHNOLOGY

Microfluidic technology is a rapidly developing field in medical diagnostics, utilizing microscale fluid systems for biological and chemical testing. This technology enables precise control of fluid flow in microscale environments and offers significant advantages over traditional diagnostic methods. In medical diagnostics, microfluidic technology has broad applications, including the rapid and accurate detection of infectious diseases, analysis of blood, urine, saliva, and other bodily fluids, as well as the detection of genetic mutations. By precisely controlling fluid flow and mixing in a controlled environment, this technology enables high-accuracy, high-sensitivity, and high-specificity analyses. It can also be integrated into portable and low-cost devices, allowing medical personnel to obtain real-time, on-site diagnostic results. Moreover, microfluidics can greatly reduce the cost and time required for diagnostic testing, making it particularly promising for use in resource-limited settings.

At the micrometer scale, fluid behavior differs significantly from that at the macroscale. In microfluidic systems, the surface area-to-volume ratio is much higher, making surface effects more dominant while inertial forces become negligible compared to viscous forces. This leads to unique fluid behaviors such as capillary flow, surface tension-driven flow, and the predominance of laminar (non-turbulent) flow.

Microfluidic immunoassay chips utilize microchannels and chambers to conduct biochemical reactions on the microscale. They detect or quantify target analytes, such as specific proteins, in biological samples using specific antibodies. The fundamental principle is as follows: a capture antibody is immobilized at a designated location within the microchannel or microchamber, referred to as the detection zone. A labeled antibody is placed near the microchannel entrance, known as the labeling zone. When a sample is introduced, the target analyte in the sample binds with the labeled antibody to form a complex. As the sample flows through the microchannel, the capture antibody selectively binds with the complex, retaining it at the detection zone. Other molecules are washed away as waste, and the label generates a detectable signal at the detection site. By comparing this signal with a calibration curve generated from known analyte concentrations, the analyte concentration in the sample can be determined.

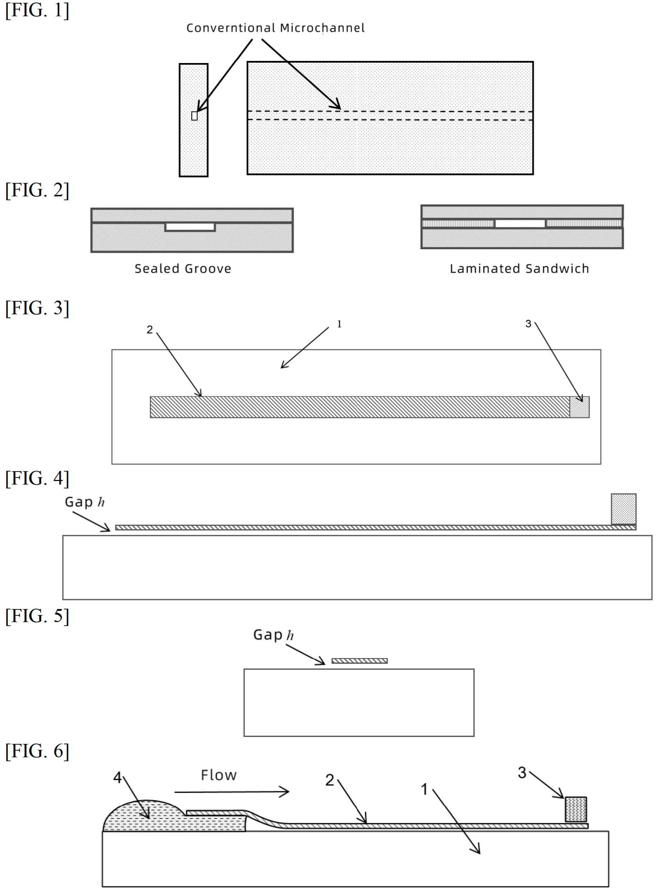

As shown in FIG. 1, typical microchannels in prior art are elongated tunnels that are sealed on the sides but open at the ends. These microchannels confine the sample fluid to a small space. Their cross-sections can be circular, square, or other shapes, with at least one dimension under 100 micrometers. Common fabrication methods include photolithography, soft lithography, micro-machining, and precision mold injection. These channels can be constructed from materials such as glass, silicon, or polymers. However, regardless of the method used-such as the sealed groove or laminated sandwich (see FIG. 2)-multi-layer bonding is required to form these narrow channels.

In immunoassay applications, microchannels often have rectangular cross-sections, with channel heights on the micron scale. Current fabrication methods pose several challenges: 1. Water molecules are only 0.28 nanometers in size, so even a 0.1 $\mu$m gap at the bonding interface can lead to leakage. Smaller gaps produce higher capillary forces, often exceeding the capillary force of the intended channel, leading to leakage. 2. The channels require micron-scale precision, demanding high mechanical and thermal stability from materials. Even small variations in adhesive thickness can change channel height, which significantly affects sample volume, concentration, flow rate, and reaction time, according to the Hagen-Poiseuille equation.3. Creating precision molds at the micron scale is extremely costly.

CONTENT OF THE INVENTION

To address the deficiencies in existing technology, the present invention proposes a microfluidic chip with a self-adjusting microchannel height.

The chip comprises a substrate and a liquid-guiding track. The liquid-guiding track is made of a strip of flexible film which is placed on the upper surface of the substrate, and the natural gap between liquid-guiding track and the substrate forms a microchannel. One end of the track is designated as the terminal end, and the other end is the starting end which serves as the sample entrance. Between the track and the substrate, a labeling zone is located near the starting end, and a detection zone is set in the middle section of the track. The microchannel is open to atmosphere along both sides of its width, the channel height can self-adjust as liquid enters.

Preferably, the substrate is made of rigid flat polymer material, and the liquid-guiding track is made of PET (polyethylene terephthalate) or PC (polycarbonate) film.

As a preferred solution, and the terminal end of the liquid-guiding track is kept in close contact with the substrate, by one of the following means:

A pressing column is anchored above the terminal end of the liquid-guiding track, which presses the terminal end of the liquid-guiding track against the surface of the substrate to keep the two in close contact with each other.

The terminal end of the liquid-guiding track is bonded to the substrate with adhesive to keep the two in close contact with each other.

The terminal end of the liquid-guiding track is weighted down by an object to keep the terminal end of the liquid-guiding track in close contact with the substrate.

Preferably, the flexible film liquid-guiding track has a thickness of 0.02-0.1 mm, a width of 2-5 mm, and a length greater than 5 times the width.

When the sample is a clear liquid (i.e. not whole blood):

Preferably, two identical, spaced, and elongated parallel slits extending in the length direction are cut in the middle of a piece of rectangular flexible film with, leaving a narrow strip film in the middle. The strip is further cut across the two slits at one end, whereat it is disjointed from the rest part of the rectangular film, hence forming a peninsula shaped liquid-guiding track.

The liquid-guiding track wherein is divided into AB, BC, CD, and DE segments in sequence along the direction of the sample flow. The BC and DE segments are parallel to the surface of the substrate. The AB segment forms an upslope, and the CD segment forms a curved downslope. The AB segment includes a sample entrance, the BC segment forms a labeling zone, and the DE segment forms a detection zone. Other parts of the flexible film are laid flat on the substrate like the DE segment, and the regions of the flexible film located before and after the DE segment and away from the marking area forms a waste reservoir.

Alternatively, a standalone elongated strip-shaped liquid-guiding track is cut out, and the liquid-guiding track is divided into AB, BC, CD, and DE segments in sequence along the flow direction of the sample liquid. The BC and DE segments are parallel to the surface of the substrate, the AB segment forms a upslope, and the CD segment forms an curved downslope; the AB segment includes a liquid entrance, the BC segment forms a labeling zone, the front of the DE segment forms a detection zone, and the rear portion forms a waste reservoir.

A cellulose membrane is laid under the waste reservoir at the terminal end of the liquid-guiding track, and the membrane thickness is less than the microchannel height *h.* The cellulose membrane extends outside the liquid-guiding track. The terminal end of liquid-guiding track and the exposed part of the cellulose membrane are tightly overlaid with an absorbent pad, and the density of the absorbent pad is greater than that of the cellulose membrane.

Preferably, the height of the BC segment is between 0.2 mm and 0.5 mm.

Preferably, the width of the AB segment is large enough to accommodate of a sample entrance with a sufficient diameter, and the width of the BC segment is small at both ends, and both sides of the two ends are cut into an inwardly concave arc.

Preferably, it further comprises a casing, which is clamped on the circumference of the substrate in the form of an upper cap, and the cross-section of the casing is U-shaped.

Preferably, the top surface of the interior of the casing protrudes downward to form a retaining ridge and a pressing column; the retaining ridge is located directly above the BC segment; the pressing column is located at the terminal end of the inner top surface of the casing, and its bottom directly presses the terminal end of the liquid-guiding track or the absorbent pad.

Preferably, a funnel-shaped sample inlet is opened on the casing directly above the AB segment, and a transparent observation window is opened on the casing directly above the detection zone.

Preferably, the bottom of the funnel-shaped sample inlet is level with the bottom of the retaining ridge, and both are spaced along the flow direction of the flexible film liquid-guiding track; the bottom of the retaining ridge is positioned close to the BC segment.

Preferably, the casing is made from plastic by injection molding.

When the sample is whole blood:

Preferably, two identical, spaced, and elongated parallel slits extending in the length direction are cut in the middle of a piece of rectangular flexible film with, leaving a narrow strip film in the middle. The strip is further cut across the two slits at one end, whereat it is disjointed from the rest part of the rectangular film, hence forming a peninsula shaped liquid-guiding track.

The liquid-guiding track is laid flat, an anticoagulant blood filtration pad is placed at the starting end of the liquid-guiding track, labeled antibody is located beneath the blood filtration pad; the starting end of the liquid-guiding track is tucked beneath the blood filtration pad, beside the labeled antibody; the liquid-guiding track also includes a detection zone and a waste reservoir along the sample liquid flow direction, with other regions of the flexible film also laid flat on the substrate to form extended waste reservoir.

Alternatively, a standalone elongated strip-shaped liquid-guiding track is cut out, The liquid-guiding track is laid flat, an anticoagulant blood filtration pad is placed at the starting end of the liquid-guiding track, labeled antibody is located beneath the blood filtration pad; the starting end of the liquid-guiding track is tucked beneath the blood filtration pad, beside the labeled antibody; the liquid-guiding track also includes a detection zone and a waste reservoir along the sample liquid flow direction.

A cellulose membrane is laid under the waste reservoir at the terminal end of the liquid-guiding track, and the membrane thickness is less than the microchannel height *h.* The cellulose membrane extends outside the liquid-guiding track. The terminal end of liquid-guiding track and the exposed part of the cellulose membrane are tightly overlaid with an absorbent pad, and the density of the absorbent pad is greater than that of the cellulose membrane.

Preferably, the blood filtration pad is a fiber material with a relatively low density, and an anticoagulant and a hemoglobin antibody are added to the blood filtration pad.

Preferably, it further comprises a casing, which is clamped on the circumference of the substrate in the form of an upper cap, and the cross-section of the casing is U-shaped.

Preferably, the top surface of the interior of the casing protrudes downward to form a retaining ridge and a pressing column; the pressing column is located at the terminal end of the inner top surface of the casing, and its bottom directly presses the terminal end of the liquid-guiding track or the absorbent pad.

Preferably, a funnel-shaped sample inlet is opened on the casing directly above the blood filtration pad, and a transparent observation window is opened on the casing directly above the detection zone.

Preferably, the bottom of the funnel-shaped sample inlet is in close contact with the top of the blood filtration pad; the bottom of retaining ridge is positioned lower than the bottom of the funnel-shaped sample inlet and higher than the bottom of the blood filtration pad; the funnel-shaped sample inlet and the retaining ridge are spaced apart in the flow direction of the flexible film liquid-guiding track.

Preferably, the casing is made from plastic by injection molding.

The microfluidic chip with self-adjusting microchannel height made with the above technical solution has the following beneficial effects:

1. The microchannel of the present invention is formed by overlaying an unbonded flexible film liquid-guiding track to a substrate. The height of the microchannel can be self-adjusted. The consistency of the microchannel height h depends only on the consistency of the thickness of the flexible film liquid-guiding track and the flatness of the substrate. Films with good thickness consistency and plastic sheets with good flatness are available in large quantities on the market at a very low price.
The microfluidic chip of the present invention can use ready-made plastic film rolls to make liquid-guiding tracks and plastic plates to make substrates. It does not require precise processing, and the casing can be made using ordinary injection molding technology. Therefore, the manufacturing cost will be lower than that of microfluidic chips made by existing groove methods and laminated sandwich methods, and the manufacturing precision requirements are low.

2. According to experiments, when using 0.05mm thick PET film and water as the sample, the height of the microchannel is about 18 microns. The microchannel of the microfluidic chip manufactured by the existing process is difficult to reach this scale, and the small height of the microchannel requires a small amount of sample. Therefore, the microfluidic chip of the present invention has great advantages in the detection of trace samples.

3. The height of the microchannel of the present invention can be self-adjusted. If the sample is a liquid with larger molecules and higher viscosity, such as blood, according to the principles of fluid mechanics, the height of the microchannel will be automatically adjusted to allow the sample liquid to flow smoothly. This cannot be achieved in a chip with a fixed height microchannel.

DESCRIPTION OF THE DRAWINGS

FIG. 1 is a cross-sectional view and a front view of a typical microfluidic channel structure in the prior art;

FIG. 2 is a cross-sectional view of a microchannel produced by groove method and laminated sandwich method in the prior art;

FIG. 3 is a top view of the microfluidic chip in Embodiment 1 of the present invention;

FIG. 4 is a cross-sectional view of FIG. 3 along the length direction of the liquid-guiding track;

FIG. 5 is a cross-sectional view of FIG. 3 along the width direction of the liquid-guiding track;

FIG. 6 is a schematic diagram of the working principle of the microfluidic chip in Embodiment 1 of the present invention (a cross-sectional view in the longitudinal direction);

FIG. 7 is a schematic diagram of the working principle of the microfluidic chip in Embodiment 1 of the present invention (a cross-sectional view in the width direction);

FIG. 8 is a schematic diagram of force balance of the microfluidic chip during operation in Embodiment 1 of the present invention;

FIG. 9 is a cross-sectional view along the length direction of the microfluidic chip in Embodiment 2 of the present invention;

FIG. 10 is a top view of the film in Embodiment 2 of the present invention;

FIG. 11 is a cross-sectional view of a substrate and a liquid-guiding track along the length direction in the Embodiment 2 of the present invention;

FIG. 12 is a top view of the casing in Embodiment 2 of the present invention;

FIG. 13 is a second cross-sectional view along the length direction of the substrate and the liquid-guiding track in the Embodiment 2 of the present invention;

FIG. 14 is a second top view of the substrate and the liquid-guiding track along the length direction in the Embodiment 2 of the present invention;

FIG. 15 is a cross-sectional view along the length direction of the microfluidic chip in Embodiment 3 of the present invention;

In the figures: substrate 1, liquid-guiding track 2, pressing column 3, sample liquid 4, labeling zone 5, detection zone 6, waste reservoir 7, sample entrance 8, casing 9, retaining ridge 10, observation window 11, sample inlet 12, labeled antibody 13, capture antibody 14, cellulose membrane 15, absorbent pad 16, blood filtration pad 17.

SPECIFIC IMPLEMENTATIONS

It should be noted that, in the absence of contradictions, the embodiments and features in the embodiments of the present invention can be combined with each other. Unless otherwise defined, the technical terms used in the following embodiments have the same meanings as those generally understood by technicians in the field to which the present invention belongs. The test reagents used in the following embodiments, unless otherwise specified, are all conventional biochemical reagents; the experimental methods, unless otherwise specified, are all conventional methods.

The present invention is further explained below in conjunction with embodiments and drawings. It should be understood that the present invention is not limited to the specific embodiments described.

Embodiment 1

As shown in FIGS. 3-5, the microfluidic chip with self-adjusting microchannel height of the present invention comprises a substrate 1 and a flexible film liquid-guiding track 2, wherein the substrate 1 is a rigid flat polymer material substrate (such as an acrylic plate); an open microchannel with self-adjusting height is overlaid on the substrate 1, and each microchannel is formed by placing an elongated liquid-guiding track 2 on the upper surface of the substrate 1; the liquid-guiding track 2 is made of a strip of flexible film, specifically a polymer material film, preferably a PET or PC film, and the thickness of the flexible film liquid-guiding track 2 does not exceed 0.1 mm (can be 0.02-0.1 mm), the width is between 2-5 mm, and the length is greater than 5 times the width;

There is a gap of height h between the liquid-guiding track 2 placed on the upper surface of the substrate 1 and the upper surface of the substrate 1 to form a microchannel of height h, and the height h is in the micrometer scale; one end of the liquid-guiding track 2 is the terminal end, and the other end naturally rest flat under the force of gravity, which is the starting end, and the starting end is where the sample entrance is; between the flexible film liquid-guiding track 2 and the substrate 1, a labeled antibody 13 is placed near the starting end, and a capture antibody 14 is placed in the middle.

As a further optimization of this embodiment, the liquid-guiding track 2 can be straight or curved in the length direction. The curved liquid-guiding track can increase the effective length of the flow channel, reduce the flow rate, and is beneficial to mixing.

As a further optimization of this embodiment, the terminal end of the liquid-guiding track 2 is kept in close contact with the substrate 1. Specific ways to doing so include but are not limited to the following three:

1. The terminal end of the liquid-guiding track 2 is fitted with a pressing column, which presses the terminal end of the liquid-guiding track against the surface of the substrate 1 to keep the two in close contact. The pressing column can be mounted on other fitting components or on the substrate 1. The pressing column can be in the shape of a cube, a prism, a ridge, etc., as long as the terminal end of the liquid-guiding track 2 can be restricted so that the relative distance between the terminal end of the liquid-guiding track 2 and the substrate 1 remains constant or varies minimally. The specific reasons will be described in the following content in conjunction with the equations.

2. The terminal end of the liquid-guiding track 2 is bonded to the substrate 1 (for example, with adhesives or thermal bonding) to keep the two in close contact.

3. A counterweight is applied to the terminal end of the liquid-guiding track 2, and the counterweight weighs down the terminal end of the liquid-guiding track 2 to keep the terminal end of the liquid-guiding track 2 in close contact with the substrate 1. The counterweight can be formed of any additional material, or can be formed by changing the thickness of the material at the terminal end of the liquid-guiding track 2, or by folding.

Taking the prism shape of the pressing column as an example, a pressing column 3 is formed, and the pressing column 3 presses one end of the flexible film liquid-guiding track 2 against the substrate 1.

The liquid-guiding track 2 of the present invention is a flexible film material, which will lie flat on the substrate 1 under the force of gravity. However, the seemingly smooth substrate 1 and the flexible film liquid-guiding track 2 are not smooth at the nanometer and micrometer scales. There will be a gap $h$ (microchannel height $h$) of 1 micron to several microns between the two, that is, the flexible film liquid-guiding track 2 lying flat on the substrate 1 under the force of gravity is not absolutely mated with the substrate 1 without gap.

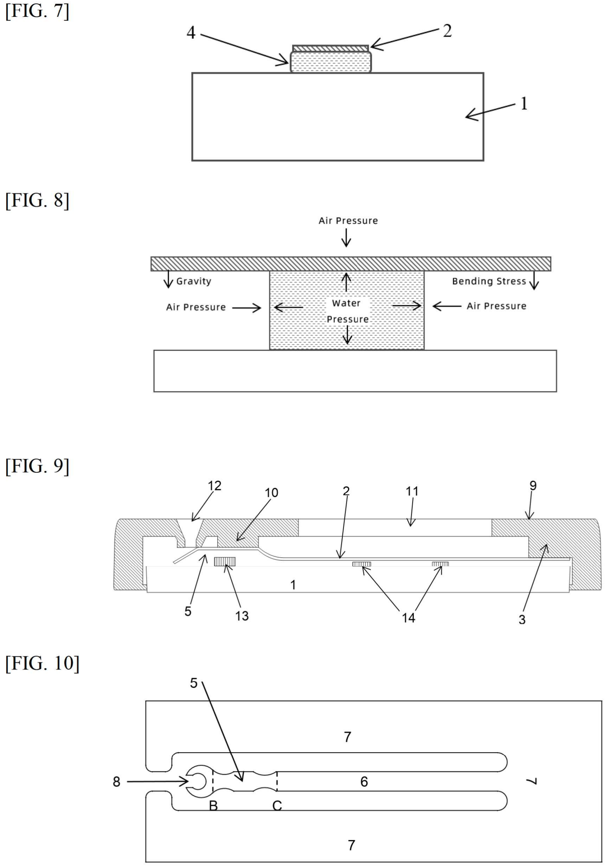

When the sample liquid 4 comes into contact with the starting end of the flexible film liquid-guiding track 2, the sample liquid 4 will be pulled into the gap $h$ by the capillary force. Since the liquid-guiding track 2 is a flexible material such as a flexible film, under the pressure generated by the capillary effect (the capillary effect will cause the water in the gap to generate pressure), the part with the sample liquid 4 will be slightly lifted up, and the terminal end of the liquid-guiding track 2 will be pressed by the top pressure column 3, and the gap at the terminal end will be kept minimum. According to the Yung-Laplace Equation, the pressure difference $\Delta P$ inside and outside the liquid interface is inversely proportional to the gap $h$ (see the following formula (1)), so the sample liquid 4 will continue to flow to the terminal end under the pull of capillary force (see FIG. 6). And because the sample liquid 4 is attracted by the adhesion force of the lower surface of the flexible film liquid-guiding track 2 (the film surface is hydrophilic, and when it comes into contact with the liquid, it will produce intermolecular attraction to the water molecules in the liquid, and there is also attraction between the liquid molecules, so the liquid will gather under the film), the sample liquid 4 will form a elongated microchannel along the lower surface of the flexible film liquid-guiding track 2 when flowing, and will not spread to the two sides (see FIG. 7).

$$\Delta P = \frac{2\,\gamma\,cos\theta}{h}\ (1)$$

Where $\gamma$ is the surface tension coefficient and $\theta$ is the contact angle between the liquid surface and the channel wall.

The stable microchannel height h must make the pressure difference between the sample liquid 4 and the atmosphere reach a equilibrium with the gravity and bending stress of the liquid-guiding track 2 (i.e., water pressure - air pressure = gravity + bending stress, see FIG. 8). Assuming that the stiffness and thickness of the flexible film liquid-guiding track 2 are proportional, the microchannel height can be derived by combining the Young-Laplace equation and Hook's Law.

$$h = \sqrt{\frac{2\gamma cos\theta \cdot l}{t \cdot \sigma} + \left(\frac{\rho \cdot g \cdot l}{2\sigma}\right)^2} - \frac{\rho \cdot g \cdot l}{2\sigma}\ (2)$$

Where, $l$ is the length of the liquid-guiding track, $t$ is the thickness of the liquid-guiding track, $\sigma$ is the stress coefficient of the liquid-guiding track, $\rho$ is the density of the liquid-guiding track, and g is the gravitational constant.

Taking partial differential of the above equation (2) can derive: $\partial h/(\partial t)<0$, $\partial h/\partial l>0$, and when $l$ is long enough, $\partial h/\partial l \rightarrow 0$. It can be seen that the microchannel height h is inversely correlated with the thickness $t$ and positively correlated with the length $l$. When $l$ is long enough, the length no longer affects the microchannel height. For a given sample liquid 4 and the properties of flexible film material of the liquid-guiding track 2, the microchannel height h is completely determined by fluid mechanics theory, and the requirements for manufacturing precision are thus no longer as high.

Embodiment 2

In order to improve the operational stability of the microfluidic chip of the Embodiment 1, the Embodiment 2 introduced solution as following:

As shown in FIGS. 9-12, the microfluidic chip with self-adjusting microchannel height of the present invention comprises a substrate 1, a piece of flexible film and a casing 9, wherein the flexible film is placed on the substrate 1, and two identical, spaced, elongated parallel slits extending in the length direction are cut out in the middle of the flexible film by a die-cutting process, wherein the width of the slits is no less than 1 mm, and a liquid-guiding track 2 is formed between the two slits, and an open microchannel with self-adjusting height is formed between the liquid-guiding track 2 and the substrate 1; the liquid-guiding track 2 is formed with three inflection points B, C, and D in sequence along the flow direction of the sample liquid 4, with the beginning and the terminal end being A, D respectively. That is, the liquid-guiding track 2 is divided into AB, BC, CD, and DE segments in sequence along the flow direction of the sample liquid 4. The BC and DE segments are parallel to the surface of the substrate 1. The AB segment forms an upslope, and the CD segment forms a curved downslope forming an arch. The center point of the arch distant from the substrate 1, that is, the BC segment is higher than the CD segment; the AB segment is includes a sample entrance 8, the BC segment forms a labeling zone 5, and the DE segment forms a detection zone. The other parts of the flexible film are laid flat on the substrate 1 like the DE segment, and the regions of the flexible film located before and after the DE segment and away from the labeling zone 5 forms a waste reservoir 7;

The casing 9 is clamped to the circumference of the substrate in the form of an upper cap. The cross-section of the casing 9 is roughly U-shaped. Along the liquid flow direction of the liquid-guiding track 2, the top surface of the inner part of the casing 9 protrudes downward to form a retaining ridge 10 and a top pressuring column 3. The retaining ridge 10 is located just above the BC segment. A funnel-shaped sample inlet 12 is opened on the casing 9 just above the AB segment. The bottom of the sample inlet 12 is level with the bottom of the retaining ridge 10, and is spaced at intervals along the diversion direction of the liquid-guiding track 2, that is, a groove is carved out between the bottom of the funnel-shaped sample inlet 12 and the retaining ridge 10, and the groove can prevent the gap between the retaining ridge 10 and the liquid-guiding track 2 from drawing in the sample liquid from the sample inlet 12; the bottom of the retaining ridge 10 is kept as close as possible to the BC segment of the liquid-guiding track; a observation window 11 is opened on the casing 9 just above the detection area 6, and the top pressuring column 3 is located at inner top surface of the casing 9 directly above the terminal end of the liquid-guiding track 2, and its bottom presses on the terminal end of the flexible film liquid-guiding track 2;

A labeled antibody 13 is placed under the labeling zone 5, and a plurality of capture antibodies 14 are placed under the detection zone 6.

As a further optimization of this embodiment, the housing 9 is a plastic housing processed by injection molding.

As a further optimization of this embodiment, the width of the AB segment is large enough to accommodate of a sample entrance with a sufficient diameter, and the width of the BC segment is small at both ends, and both sides of the two ends are cut into an inwardly concave arc.

Liquid flowing in a channel with a height of less than 100 microns behaves as laminar flow, which is smooth but not conducive to mixing. Because the labeled antibody 13 needs to be fully mixed with the sample liquid, the labeling area 5 cannot be a microfluidic channel. The labeling zone 5 needs to be designed with a channel height between 200 microns and 0.5 mm. The labeled antibody 13 is also an obstacle in the channel. When the sample liquid flows into the obstacle, a vortex will be generated, which helps the sample liquid to mix with the dissolved labeled antibody.

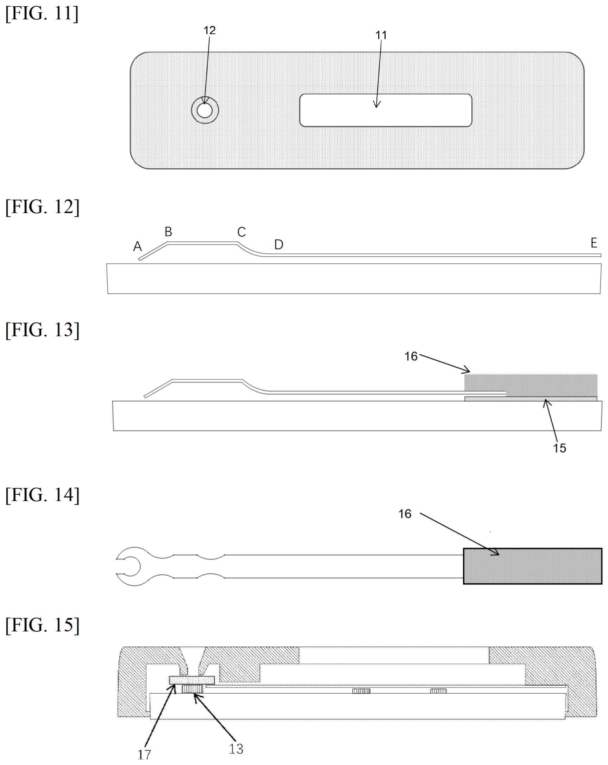

As shown in FIGS. 13-14, in order to improve the waste liquid collection, a novel design of waste reservoir is presented. First, an standalone liquid-guiding track 2 is made, and an open microchannel with self-adjustable height is formed between the liquid-guiding track 2 and the substrate 1; the liquid-guiding track 2 is formed with three inflection points B, C, and D in sequence along the flow direction of the sample liquid 4, with the beginning and the terminal end being A, D respectively. That is, the liquid-guiding track 2 is divided into AB, BC, CD, and DE segments in sequence along the flow direction of the sample liquid 4. The BC and DE segments are parallel to the surface of the substrate 1. The AB segment forms an upslope, and the CD segment forms a curved downslope forming an arch. The center point of the arch distant from the substrate 1, that is, the BC segment is higher than the CD segment; the AB segment includes a sample entrance 8, the BC segment forms a labeling zone 5, the front of the DE segment forms a detection zone, and the rear forms a waste reservoir.

A cellulose membrane 15 is laid under the waste reservoir at the terminal end of the flexible film liquid-guiding track 2, and the membrane's thickness is less than the microchannel height *h*. The cellulose membrane 15 extends outside the liquid-guiding track 2. and absorbent pad 16 is pressed on the terminal end of the flexible film liquid-guiding track 2 and the exposed cellulose membrane 15, and the density of the absorbent pad is greater than that of the cellulose membrane. The absorbent pad 16 is located at the bottom of the top pressuring column 3. When the thickness of the absorbent pad 16 is large enough, it can also play the role of the top pressing column, or the thickness of the absorbent pad is directly set to reach the top surface of the casing 9. This method requires two more procedures, but the waste reservoir can holed a large amount of water, and the chip can be made slimer, which is suitable for chips that require a large amount of sample.

Embodiment 3

The third embodiment is particularly suitable for the case where the sample liquid is whole blood. When the sample liquid is whole blood it needs to be anticoagulated first and the filter out the red blood cells. Therefore, the sample addition and labeling methods need to be adjusted. As shown in FIG. 15, the substrate 1 and the casing 9 (except for the bottom height of the retaining ridge) of the Embodiment 3 are arranged in the same manner as those of the Embodiment 2. The arrangement of the liquid-guiding track 2 is adjusted. The arrangement of the waste reservoir and the detection zone is the same as that of the Embodiment 2 (both waste reservoir designs are acceptable). The only difference from the Embodiment 2 is that the liquid-guiding track 2 is not bent along the flow direction of the sample liquid 4 but is laid flat on the substrate 1. An anticoagulant blood filtration pad 17 is placed below a funnel-shaped sample inlet 12, and a labeled antibody 13 is placed blow of the anticoagulant blood filtration pad 17. The anticoagulant blood filtration pad 17 and the labeled antibody 13 are vertically aligned in the center, and the circumferential size of the blood filtration pad cotton 17 is larger than the labeled antibody 13. The circumferential range of the anticoagulant blood filter 17 is smaller than the retaining ridge 10, and does not exceed the groove between the bottom of the sample inlet 12 and the retaining ridge 10; the starting end of the liquid guide track 2 is located below the anticoagulant blood filtration pad 17 and besides the labeled antibody 13. The bottom of the funnel-shaped sample inlet is in close contact with the upper part of the blood filtration pad, the bottom of the retaining ridge is lower than the bottom of the sample inlet and higher than the bottom of the anticoagulant blood filtration pad, and the bottom of the retaining ridge is kept at a distance of about 0.1 mm from the BC section of the liquid-guiding track. The anticoagulant blood filtration pad is a fiber material with a low density. Anticoagulant and hemoglobin antibody are added to the blood-filtration pad. After blood filtration pad becomes supersaturated with blood sample, plasma will precipitate from the bottom. The plasma and the labeled antibody are mixed and pulled into the liquid-guiding track 2 by the capillary force.

The above technical solutions only reflect the preferred configurations of the present invention. Minor modifications that may be made to certain parts thereof by technicians in this technical field all reflect the principles of the present invention and fall within the scope of protection of the present invention.

**Claims**

1. A microfluidic chip with self-adjustable microchannel height, **characterized in that** it comprises a substrate and a liquid-guiding track, wherein a microchannel with self-adjustable height is overlaid on the substrate, wherein the microchannel is formed by placing an elongated liquid-guiding track on the upper surface of the substrate, wherein the liquid-guiding track is made of a strip of flexible film, and both sides of the liquid-guiding track are open in the width direction; one end of the liquid-guiding track is the terminal end, and the other end is the starting end for sample addition; a labeling zone is located near the starting end, and a detection zone is located in the middle section between the liquid-guiding track and the substrate.

2. The microfluidic chip of claim 1, wherein the terminal end of the liquid-guiding track is kept in close contact with the substrate.

3. The microfluidic chip of claim 2, wherein the terminal end of the liquid-guiding track is fitted with a pressing column, and the pressing column presses the terminal end of the liquid-guiding track against the surface of the substrate to keep the two in close contact.

4. The microfluidic chip of claim 2, wherein the terminal end of the liquid-guiding track is bonded to the substrate to keep the two in close contact.

5. The microfluidic chip of claim 2, wherein a counterweight is applied to the terminal end of the liquid-guiding track, the counterweight weighs down the terminal end of the liquid-guiding track to keep the terminal end of the liquid-guiding track in close contact with the substrate.

6. The microfluidic chip of claim 3, wherein the flexible film liquid-guiding track has a thickness of 0.02-0.1 mm, width of 2-5 mm, and a length greater than 5 times its width.

7. The microfluidic chip of claim 3 or 6, wherein two spaced elongated slits are cut along the length of a piece of flexible film, forming a liquid-guiding track in between; the slits do not extend across the entire film length.

8. The microfluidic chip of claim 7, wherein the liquid-guiding track is divided into AB, BC, CD, and DE segments in sequence along the direction of sample flow; BC and DE segments are parallel to the substrate surface, AB segment forms an upward slope, and CD segment forms a curved downward slope; AB segment includes a sample entrance, BC segment forms the labeling zone, DE segment forms the detection zone, and other parts of the flexible film are laid flat on the substrate in the same manner as the DE segment, and the regions of the flexible film located before and after the DE segment and away from the labeling zone forms form the waste reservoir.

9. The microfluidic chip of claim 3 or 6, wherein the flexible film liquid-guiding track is a standalone strip, and the liquid-guiding track is divided into AB, BC, CD, and DE segments in sequence along the direction of sample flow; BC and DE segments are parallel to the substrate surface, AB segment forms an upward slope, and CD segment forms a curved downward slope; AB segment includes a sample entrance, BC segment forms the labeling zone, the front of the DE segment forms the detection zone, and the rear portion forms the waste reservoir.

10. The microfluidic chip of claim 9 wherein a cellulose membrane is laid under the waste reservoir at the terminal end of the flexible film liquid-guiding track, the membrane thickness is less than the microchannel height h, the cellulose membrane extends outside the flexible film liquid-guiding track, and absorbent pad is pressed on the terminal end of the flexible film liquid-guiding track and the exposed cellulose membrane, and the density of the absorbent pad is greater than that of the cellulose membrane.

11. The microfluidic chip of any of claims 3, 6, 8, or 10, further comprising an external casing that is clamped on the circumference of the substrate in the form of upper cap, with a U-shaped cross-section.

12. The microfluidic chip of claim 11, wherein the top surface of the interior of the casing protrudes downward to form a retaining ridge and a pressing column; the retaining ridge is located directly above the BC segment; the pressing column is located at the terminal end of the inner top surface of the casing, and its bottom directly presses the terminal end of the flexible film liquid-guiding track or the absorbent pad.

13. The microfluidic chip of claim 12, wherein a funnel-shaped sample inlet is opened on the casing directly above the AB segment, and a transparent observation window is opened on the casing directly above the detection zone.

14. The microfluidic chip of claim 13, wherein the bottom of the funnel-shaped sample inlet is level with the bottom of the retaining ridge, and both are spaced along the flow direction of the flexible film liquid-guiding track; the bottom of the retaining ridge is positioned close to the BC segment.

15. The microfluidic chip of claim 7, wherein the flexible film liquid-guiding track is laid flat, and an anticoagulant blood filtration pad is placed at the starting end of the flexible film liquid-guiding track; labeled antibody is located beneath the blood filtration pad; the starting end of the flexible film liquid-guiding track is tucked beneath the blood filtration pad, beside the labeled antibody; the flexible film liquid-guiding track also includes a detection zone and a waste reservoir along the sample liquid flow direction, with other regions of the flexible film also laid flat on the substrate to form extended waste reservoir.

16. The microfluidic chip of claim 3 or 6, wherein the flexible film liquid-guiding track is a standalone strip; wherein the flexible film liquid-guiding track is laid flat, and an anticoagulant blood filtration pad is placed at the starting end of the flexible film liquid-guiding track; labeled antibody is located beneath the blood filtration pad; the starting end of the flexible film liquid-guiding track is tucked beneath the blood filtration pad, beside the labeled antibody; the flexible film liquid-guiding track also includes a detection zone and a waste reservoir along the sample liquid flow direction.

17. The microfluidic chip of claim 16 wherein a cellulose membrane is laid under the waste reservoir at the terminal end of the flexible film liquid-guiding track, the membrane thickness is less than the microchannel height h, the cellulose membrane extends outside the flexible film liquid-guiding track, and absorbent pad is pressed on the terminal end of the flexible film liquid-guiding track and the exposed cellulose membrane, and the density of the absorbent pad is greater than that of the cellulose membrane.

18. The microfluidic chip of claim 15 or 17, wherein the anticoagulant blood filtration pad is made of low-density fibrous material, wherein anticoagulant and hemoglobin antibodies are applied to the blood filtration pad.

19. The microfluidic chip of claim 15 or 17, further comprising an external casing that is clamped on the circumference of the substrate in the form of upper cap, with a U-shaped cross-section.

20. The microfluidic chip of claim 19, wherein the top surface of the interior of the casing protrudes downward to form a retaining ridge and a pressing column; the pressing column is located at the terminal end of the inner top surface of the casing, and its bottom directly presses the terminal end of the flexible film liquid-guiding track or the absorbent pad.

21. The microfluidic chip of claim 20, wherein a funnel-shaped sample inlet is opened on the casing directly above the blood filtration pad, and a transparent observation window is opened on the casing directly above the detection zone.

22. The microfluidic chip of claim 21, wherein the bottom of the funnel-shaped sample inlet is in close contact with the top of the blood filtration pad; the bottom of retaining ridge is positioned lower than the bottom of the funnel-shaped sample inlet and higher than the bottom of the blood filtration pad; the funnel-shaped sample inlet and the retaining ridge are spaced apart in the flow direction of the flexible film liquid-guiding track.

**Amended claims under Art. 19.1 PCT**

1. A microfluidic chip with self-adjustable microchannel height, **characterized in that** the microfluidic chip comprises a substrate and a liquid-guiding track, wherein a microchannel with self-adjustable height is overlaid on the substrate, wherein the microchannel is formed by placing an elongated liquid-guiding track on an upper surface of the substrate, wherein the liquid-guiding track is made of a strip of flexible film, and both sides of the liquid-guiding track are open in a width direction; one end of the liquid-guiding track is a terminal end, and the other end of the liquid-guiding track is a starting end for sample addition; a labeling zone is located near the starting end, and a detection zone is located in a middle section between the liquid-guiding track and the substrate.

2. The microfluidic chip according to claim 1, wherein the terminal end of the liquid-guiding track is kept in close contact with the substrate.

3. The microfluidic chip according to claim 2, wherein the terminal end of the liquid-guiding track is fitted with a pressing column, and the pressing column presses the terminal end of the liquid-guiding track against the surface of the substrate to keep the two in close contact.

4. [Amended] The microfluidic chip according to claim 1,2 or 3, wherein the flexible film liquid-guiding track has a thickness of 0.02-0.1 mm, width of 2-5 mm, and a length greater than 5 times its width.

5. [Amended] The microfluidic chip according to claim 1,2 or 3, wherein two spaced elongated slits are cut along a length of a piece of flexible film, forming a liquid-guiding track in between; the slits do not extend across an entire film length.

6. [Amended] The microfluidic chip according to claim 5, wherein the liquid-guiding track is divided into AB, BC, CD, and DE segments in sequence along a direction of sample flow; the BC and DE segments are parallel to a substrate surface, the AB segment forms an upward slope, and the CD segment forms a curved downward slope; the AB segment comprises a sample entrance, the BC segment forms the labeling zone, the DE segment forms the detection zone, and other parts of the flexible film are laid flat on the substrate in the same manner as the DE segment, and regions of the flexible film located before and after the DE segment and away from the labeling zone form the waste reservoir.

7. [Amended] The microfluidic chip according to claim 1,2 or 3, wherein a flexible film liquid-guiding track is a standalone strip, and the liquid-guiding track is divided into AB, BC, CD, and DE segments in sequence along a direction of sample flow; the BC and DE segments are parallel to a substrate surface, the AB segment forms an upward slope, and the CD segment forms a curved downward slope; the AB segment comprises a sample entrance, the BC segment forms the labeling zone, a front of the DE segment forms the detection zone, and a rear portion forms a waste reservoir.

8. [Amended] The microfluidic chip according to claim 7, wherein a cellulose membrane is laid under the waste reservoir at the terminal end of the flexible film liquid-guiding track, a membrane thickness is less than a microchannel height h, the cellulose membrane extends outside the flexible film liquid-guiding track, and absorbent pad is pressed on the terminal end of the flexible film liquid-guiding track and an exposed cellulose membrane, and a density of the absorbent pad is greater than a density of the cellulose membrane.

9. [Amended] The microfluidic chip according to claims 1,2,3, 6 or 8, further comprising a casing that is clamped on a circumference of the substrate in form of upper cap, with a U-shaped cross-section.

10. [Amended] The microfluidic chip according to claim 9, wherein a top surface of an interior of the casing protrudes downward to form a retaining ridge and a pressing column; the retaining ridge is located directly above the BC segment; the pressing column is located at a terminal end of an inner top surface of the casing, and its bottom directly presses the terminal end of the flexible film liquid-guiding track or the absorbent pad.

11. [Amended] The microfluidic chip according to claim 10, wherein a funnel-shaped sample inlet is opened on the casing directly above the AB segment, and a transparent observation window is opened on the casing directly above the detection zone.

12. [Amended] The microfluidic chip according to claim 11, wherein a bottom of the funnel-shaped sample inlet is level with a bottom of the retaining ridge, and both are spaced along a flow direction of the flexible film liquid-guiding track; the bottom of the retaining ridge is positioned close to the BC segment.

13. [Amended] The microfluidic chip according to claim 5, wherein a flexible film liquid-guiding track is laid flat, and an anticoagulant blood filtration pad is placed at the starting end of the flexible film liquid-guiding track; labeled antibody is located beneath the blood filtration pad; the starting end of the flexible film liquid-guiding track is tucked beneath the blood filtration pad, beside the labeled antibody; the flexible film liquid-guiding track also comprises a detection zone and a waste reservoir along a sample liquid flow direction, with other regions of the flexible film also laid flat on the substrate to form extended waste reservoir.

14. [Amended] The microfluidic chip according to claim 1,2 or 3, wherein a flexible film liquid-guiding track is a standalone strip; wherein the flexible film liquid-guiding track is laid flat, and an anticoagulant blood filtration pad is placed at the starting end of the flexible film liquid-guiding track; labeled antibody is located beneath the blood filtration pad; the starting end of the flexible film liquid-guiding track is tucked beneath the blood filtration pad, beside the labeled antibody; the flexible film liquid-guiding track also comprises a detection zone and a waste reservoir along a sample liquid flow direction.

15. [Amended] The microfluidic chip according to claim 14, wherein a cellulose membrane is laid under the waste reservoir at the terminal end of the flexible film liquid-guiding track, a membrane thickness is less than a microchannel height h, the cellulose membrane extends outside the flexible film liquid-guiding track, and absorbent pad is pressed on the terminal end of the flexible film liquid-guiding track and an exposed cellulose membrane, and a density of the absorbent pad is greater than a density of the cellulose membrane.

16. [Amended] The microfluidic chip according to claim 13 or 15, wherein the anticoagulant blood filtration pad is made of low-density fibrous material, wherein anticoagulant and hemoglobin antibodies are applied to the blood filtration pad.

17. [Amended] The microfluidic chip according to claim 13 or 15, further comprising a casing that is clamped on a circumference of the substrate in form of upper cap, with a U-shaped cross-section.

18. [Amended] The microfluidic chip according to claim 17, wherein a top surface of an interior of the casing protrudes downward to form a retaining ridge and a pressing column; the pressing column is located at a terminal end of an inner top surface of the casing, and its bottom directly presses the terminal end of the flexible film liquid-guiding track or the absorbent pad.

19. [Amended] The microfluidic chip according to claim 18, wherein a funnel-shaped sample inlet is opened on the casing directly above the blood filtration pad, and a transparent observation window is opened on the casing directly above the detection zone.

20. [Amended] The microfluidic chip according to claim 19, wherein a bottom of the funnel-shaped sample inlet is in close contact with a top of the blood filtration pad; a bottom of the retaining ridge is positioned lower than the bottom of the funnel-shaped sample inlet and higher than a bottom of the blood filtration pad; the funnel-shaped sample inlet and the retaining ridge are spaced apart in the flow direction of the flexible film liquid-guiding track.

[FIG. 1]

Converntional Microchannel

[FIG. 2]

Sealed Groove

Laminated Sandwich

[FIG. 3]

2　　　　　　　　1　　　　　　　　3

[FIG. 4]

Gap *h*

[FIG. 5]

Gap *h*

[FIG. 6]

4　　Flow　　2　　1　　3

[FIG. 7]

[FIG. 8]

[FIG. 9]

[FIG. 10]

[FIG. 11]

[FIG. 12]

[FIG. 13]

[FIG. 14]

[FIG. 15]

## INTERNATIONAL SEARCH REPORT

| International application No. |
| --- |
| **PCT/CN2023/140451** |

| A. | CLASSIFICATION OF SUBJECT MATTER |
| --- | --- |

G01N 33/72(2006.01)i; G01N 33/53(2006.01)i; B01L 3/00(2006.01)i

According to International Patent Classification (IPC) or to both national classification and IPC

| B. | FIELDS SEARCHED |
| --- | --- |

Minimum documentation searched (classification system followed by classification symbols)

IPC: G01N B01L

Documentation searched other than minimum documentation to the extent that such documents are included in the fields searched

Electronic data base consulted during the international search (name of data base and, where practicable, search terms used)

CNTXT; ENTXTC; WPABSC; VEN; ENTXT; CJFD; CNKI; ISI Web of Science: 薛山, 高度, 宽度, 直径, 半径, 调, 改, 变, 液, 流, 微流控, 抗体; height, breadth, width, diameter, chang+, adjust+, fluid, liquid, microflow, antibod+

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 116125079 A (TIANJIN YUNJI HEALTH TECHNOLOGY CO., LTD.) 16 May 2023 (2023-05-16) <br> claims 1-18, description, paragraphs [0001]-[0061], and figures 4-15 | 1-22 |
| A | KR 20100104394 A (LG INNOTEK CO., LTD.) 29 September 2010 (2010-09-29) <br> description, paragraphs [0001]-[0049], and figures 1-5 | 1-22 |
| A | WO 2020067716 A1 (KOREA ADVANCED INSTITUTE OF SCIENCE AND TECHNOLOGY) 02 April 2020 (2020-04-02) <br> entire document | 1-22 |
| A | WO 03015923 A1 (BIOMICRO SYSTEMS, INC. et al.) 27 February 2003 (2003-02-27) <br> entire document | 1-22 |
| A | CN 111282605 A (SOOCHOW UNIVERSITY) 16 June 2020 (2020-06-16) <br> entire document | 1-22 |
| A | JP H10319018 A (EIKEN CHEMICAL CO., LTD.) 04 December 1998 (1998-12-04) <br> entire document | 1-22 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **10 January 2024** | **29 January 2024** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/140451** |

| C. | DOCUMENTS CONSIDERED TO BE RELEVANT | |
| --- | --- | --- |
| Category* | Citation of document, with indication, where appropriate, of the relevant passages | Relevant to claim No. |
| A | US 2009060791 A1 (AIDA ENGINEERING, LTD.) 05 March 2009 (2009-03-05)<br>entire document | 1-22 |

Form PCT/ISA/210 (second sheet) (July 2022)

International application No.

**PCT/CN2023/140451**

| Patent document cited in search report | | | Publication date (day/month/year) | Patent family member(s) | Publication date (day/month/year) |
|---|---|---|---|---|---|
| CN | 116125079 | A | 16 May 2023 | None | |
| KR | 20100104394 | A | 29 September 2010 | None | |
| WO | 2020067716 | A1 | 02 April 2020 | None | |
| WO | 03015923 | A1 | 27 February 2003 | None | |
| CN | 111282605 | A | 16 June 2020 | None | |
| JP | H10319018 | A | 04 December 1998 | None | |
| US | 2009060791 | A1 | 05 March 2009 | None | |

Form PCT/ISA/210 (patent family annex) (July 2022)